# EUROPÄISCHE PATENTSCHRIFT

(11) **EP 2 853 279 B1**
(45) Veröffentlichungstag und Bekanntmachung des Hinweises auf die Patenterteilung: **08.08.2018**
(21) Anmeldenummer: 14186142.7
(22) Anmeldetag: 24.09.2014
(51) Int. Cl.: A61M 16/04, A61F 13/12

(54) **Verschluss für ein Tracheostoma**
Closure for a tracheostoma
Fermeture pour trachéotomie

(30) Priorität: 27.09.2013 DE 202013104415 U; 27.09.2013 DE 102013110694; 07.04.2014 DE 202014101628 U
(43) Veröffentlichungstag der Anmeldung: 01.04.2015
(73) Patentinhaber: BMV Bender Medical Vertrieb GmbH, 53804 Much (DE)
(72) Erfinder: Bender, Thomas, 53819 Neunkirchen-Seelscheid (DE); Schnüll, Katja, 26802 Neermoor (DE)
(74) Vertreter: Lenzing Gerber Stute

(56) Entgegenhaltungen:
- EP-A2- 1 454 601
- DE-A1- 3 524 126
- DE-A1- 10 148 572
- US-A1- 2009 025 730
- US-A1- 2013 047 993
- US-A1- 2013 096 520
- US-A1- 2013 192 603

## Beschreibung

Die vorliegende Erfindung betrifft eine Vorrichtung zum temporären Verschließen eines Trachoestomas eines Patienten mit mindestens einem Mittel zum Halten der Vorrichtung am Hals des Patienten.

Eine Tracheotomie ist eine chirurgische Maßnahme, mit dem ein Zugang zur Luftröhre eines Patienten gesetzt wird, um beispielsweise eine Trachealkanüle einzusetzen. Hierdurch entsteht ein Tracheostoma. Operationsmethoden sind beispielsweise eine perkutane Punktions- und Dilatationstracheotomie, bei der nur ein vergleichsweise kleines Tracheostoma erzeugt wird, oder eine chirurgische Tracheotomie, bei der ein übersichtlicher Zugang zur Luftröhre geschaffen wird.

In vielen Fällen wird der Zugang zur Luftröhre nach beispielsweise einem operativen Eingriff nicht mehr benötigt. Ein Tracheostoma kann sich unter Umständen von allein verschließen und verheilen. In den Fällen, in denen dies nicht gelingt oder von vorneherein nicht möglich ist, muss das Tracheostoma chirurgisch geschlossen werden.

Bevor das Tracheostoma sich wieder verschließt oder operativ geschlossen wird, kann es notwendig oder zumindest sinnvoll sein zu prüfen, ob der Patient bei verschlossenem Tracheostoma beschwerdefrei Atmen kann. Hierzu wird das Tracheostoma mittels einer Vorrichtung dicht verschlossen. Auch ist es sinnvoll, ein Austreten von Luft durch das Stoma beim Atmen, Sprechen oder Husten zu verhindern, da der Luftaustritt ein eigenständiges Zusammenwachsen des Tracheostomas behindern kann. Dies kann ebenfalls mit einer Vorrichtung zum temporären Verschließen des Tracheostomas erfolgen.

Eine hierfür geeignete Vorrichtung ist aus der US 2009/0025730 A1 bekannt. Sie weist eine gewölbte Platte auf, die mit einem Band, das durch seitliche Ösen der Platte geführt ist, am Hals des Patienten befestigt wird. Auf der proximalen, also dem Tracheostoma zugewandten Seite der Platte ist ein ringförmiges Kissen vorgesehen, das an der Platte temporär oder dauerhaft angeklebt ist und das Tracheostoma nicht unmittelbar kontaktiert, sondern die Haut um das Tracheostoma herum. Allerdings kann es vorkommen, dass diese Vorrichtung das Tracheostoma nicht optimal abdeckt, so dass ein Verschluss des Tracheostomas nicht immer sicher gewährleistet ist, beispielsweise, weil die Platte nicht richtig an das Tracheostoma angesetzt wird oder sich die Ringform des Kissens nicht optimal an die das Tracheostoma umgebende Haut anlegt.

Ferner ist aus der EP 1 454 601 A2 eine Anordnung zur Stabilisierung eines Tracheostoma mit einem Stabilisierungsring bekannt, welcher äußerlich an einem Tracheostoma anliegt und zumindest teilweise in das Tracheostoma hineinragt. Zur Abdichtung des Stabilisierungsrings gegen den Rand des Tracheostoma wird auf der dem Tracheostoma zugewandten Seite der Vorrichtung ein Auflagerkissen vorgeschlagen, welches über ein geeignetes Ventil mit Luft oder Wasser befüllt werden kann.

Weiterhin ist aus der DE 101 48 572 A1 ein Ventil zum Einsetzen in ein Stomapflaster (oder beispielsweise auch in einen Stabilisierungsring wie oben beschrieben) bekannt. Es besteht aus einem kurzen Rohrstück und einer innerhalb des Rohrstücks symmetrisch bezüglich einer Rohrmittelachse angeordneten, durch mindestens eine elastische Wandung begrenzten Kammer, wobei die Kammer durch Zuführen bzw. Entnehmen eines Druckmediums in ihrem Volumen veränderbar ist, und zwar zwischen einer Öffnungsstellung mit einem minimalen Volumen, in der die Kammer einen maximalen Strömungsquerschnitt durch das Rohrstück freilässt, und einer Verschlussstellung mit einem maximalen Volumen, in der der Strömungsquerschnitt durch das Rohrstück gleich null ist.

Die Aufgabe der vorliegenden Erfindung besteht darin, eine Vorrichtung der eingangs genannten Art bereitzustellen, die ein sicheres Abdichten des Tracheostomas ermöglicht.

Diese Aufgabe wird gelöst mit einer Vorrichtung mit den Merkmalen des Anspruchs 1.

Mit einem aufblasbaren Luftkissen ist es in Verbindung mit dem Mittel zum Halten der Vorrichtung am Hals des Patienten möglich, den Druck des Kissens auf die ein Tracheostoma umgebende Haut eines Patienten gezielt so anzupassen, dass das Tracheostoma selbst bei Husten sicher verschlossen bleibt.

Es ist denkbar, dass das Luftkissen auf seiner proximalen Seite eine formstabile Kappe aufweist, deren Form an die Form der an das Tracheostoma angrenzenden Haut angepasst ist, so dass sich die Kappe gegen die Haut am äußeren Rand des Tracheostomas abstützt. Ist die Oberfläche der Kappe elastisch, kann ein dichter Verschluss des Tracheostomas gewährleistet werden, wobei der Anpressdruck durch den Druck im Luftkissen angepasst wird.

Vorzugsweise ist aber das Luftkissen an seiner proximalen, also der beim Anlegen der Vorrichtung an den Hals des Patienten dem Tracheostoma zugewandten Seite flexibel ausgebildet, so dass sich die Form des Luftkissens an die Form der an das Tracheostoma angrenzenden Haut anpassen kann. Dabei wirkt der Druck des Luftkissens an allen Kontaktstellen zur Haut gleichmäßig. Gleichzeitig passt sich das Kissen an die Form der Haut um das Tracheostoma herum und damit an die Größe des Tracheostomas an. Damit bietet die Vorrichtung für eine Vielzahl von Tracheostomas unterschiedlicher Form und Größe einen sicheren, luftdichten Verschluss.

In einer bevorzugten Ausgestaltung der Erfindung hat das Luftkissen einen runden, insbesondere kreisrunden, Querschnitt. Hierdurch ist die Grundform des Luftkissens optimal an die Form einer Vielzahl von verschließenden Stomata angepasst. Es sind aber auch andere Querschnittsformen, beispielsweise Ovale, möglich. Bei einer sehr elastischen bzw. flexiblen Außenhaut des Luftkissens ist auch ein eckiger Querschnitt denkbar, da sich die Ecken des Querschnitts nicht in der aufgeblasenen flexiblen bzw. elastischen Kissenhülle fortsetzen.

In einer weiteren bevorzugten Ausführungsform der Erfindung ist an der distalen, also der beim Anlegen der Vorrichtung an den Hals des Patienten vom Tracheostoma abgewandten Seite des Luftkissens eine mit einem Ventil verschließbare Zuleitung für Luft oder ein anderes Fluid vorgesehen ist, über die das Luftkissen aufgeblasen bzw. befüllt werden kann. Das Ventil kann als Rückschlagventil, aber auch manuell betätigbar ausgebildet sein. Ein manuell betätigbares Ventil, mit dem Luft aus dem Luftkissen kontrolliert abgelassen werden kann, kann von Vorteil sein, da es durch das kontrollierte Ablassen von Luft möglich ist, den Anpressdruck des an einem Tracheostoma anliegenden, aufgeblasenen Luftkissens gezielt einzustellen. Das Ventil kann auch so ausgebildet sein, dass es durch Einsetzen der Düse einer Spritze öffnet, so dass mit der Spritze gezielt Luft in das Luftkissen hineingedrückt oder herausgesogen werden kann.

In einer weiteren Variante der Erfindung kann das Luftkissen selbstaufblasbar ausgebildet sein, beispielsweise in dem innerhalb des Luftkissens ein elastischer, offenzelliger Schaumstoff enthalten ist, der zunächst zusammengedrückt ist und der sich nach Öffnen eines Ventils innerhalb der Wandung des Luftkissens ausdehnen kann. Erfolgt dies, wenn das Luftkissen bereits an das Tracheostoma angelegt ist, kann sich das Luftkissen an die Form der an das Tracheostoma angrenzenden Haut anpassen.

Auf seiner distalen Seite ist das Luftkissen vorzugsweise steif ausgebildet. So ist es möglich, die Position des Luftkissens am Tracheostoma mit den Haltemitteln genau zu fixieren. Insbesondere dann, wenn in einer weiteren bevorzugten Ausgestaltung der Erfindung mindestens ein Heftpflaster als Haltemittel vorgesehen ist, bietet ein steifer Teil des Luftkissens eine gute Haftfläche für das Heftpflaster. Wäre die Haftfläche am Kissen flexibel und insbesondere elastisch, könnte sich das Heftpflaster möglicherweise bei Ausdehnen der Haftfläche lösen.

Das Pflaster weist vorzugsweise an mindestens zwei gegenüberliegenden Seiten des Luftkissens jeweils eine sich vom Kissen weg erstreckende Lasche auf. Damit kann das Luftkissen sicher am Hals befestigt werden. Anders als bei einer Befestigung des Luftkissens über ein Band wirken die Haltekräfte des Haftpflasters unmittelbar dem vom Luftkissen aufgebrachten Druck entgegen. Außerdem verrutscht ein einmal auf die Haut aufgeklebtes Pflaster nicht mehr ohne weiteres. So kann das Luftkissen deutlich besser zum Verschließen des Tracheostomas in Position gehalten werden. Bevorzugt sind dabei mindestens drei Laschen vorgesehen, die in Umfangsrichtung des Luftkissens um 90° zueinander versetzt angeordnet sind. Insbesondere wenn eine der Laschen, die bei einem an das Tracheostoma angelegten Luftkissen in Längsrichtung des Halses verläuft, vergleichsweise lang ausgebildet ist, kann diese bis unter das Kinn des Patienten am Hals angeklebt werden. Hiermit kann ein ganz besonders sicherer Halt des Luftkissens in seiner Position gewährleistet werden.

Zusätzlich kann die erfindungsgemäße Vorrichtung mindestens eine Leitung aufweisen, die im Bereich des Luftkissens von der distalen Seite der Vorrichtung zu seiner proximalen Seite geführt ist, und mit der ein Fluid von der proximalen Seite des Luftkissens abgeführt werden kann. So ist es möglich, ohne die Mittel zum Halten des Pflasters zu lösen, Wundsekret abzusaugen, beispielsweise dann, wenn ein tracheotomierter Patient nach einer Operation noch nicht kräftig genug ist, sich bildendes Wundsekret abzuhusten. Es ist auch denkbar, dem operierten Tracheostoma über eine solche Leitung Wirkstoffe zur Wundversorgung zuzuführen.

In einer einfachen und insoweit bevorzugten Ausführungsform ist die Leitung mit einem durch einen flanschartigen Rand des Luftkissens hindurchgeführten Kanalstück und einem sich daran anschließenden Schlauchstück gebildet. Das Schlauchstück ist dabei vorteilhafterweise lösbar mit dem Kanalstück verbunden, und/oder es ist am freien Ende des Schlauchstücks ein Anschluss zum Verbinden des Schlauchstücks mit einer Vorrichtung zum Erzeugen eines Unterdrucks, insbesondere eine Spritze oder eine Pumpe, vorgesehen. Im Folgenden wird die Erfindung anhand von Figuren, in denen ein bevorzugtes erfindungsgemäßes Ausführungsbeispiel dargestellt ist, näher erläutert.

Es zeigen:
- Figur 1: eine Aufsicht auf die erfindungsgemäße Verschlussvorrichtung;
- Figur 2: eine Schnittansicht durch die in Figur 1 dargestellte Verschlussvorrichtung;
- Figur 3: eine Aufsicht einer ähnlichen erfindungsgemäßen Verschlussvorrichtung; und
- Figur 4: eine Schnittansicht durch die in Figur 3 dargestellte Verschlussvorrichtung.

Die erfindungsgemäße Vorrichtung zum temporären Verschließen eines Tracheostomas besteht im Wesentlichen aus einem Luftkissen 1 und ein dieses haltende Heftpflaster 2.

Das Luftkissen 1 weist auf seiner distalen, also der bei Anlegen der Vorrichtung an den Hals eines Patienten vom Tracheostoma abgewandten Seite eine vergleichsweise steife, kreisrunde Basisplatte 3 auf, auf dessen proximaler Seite eine flexible, folienartige Schicht 4 aufgebracht ist. Die folienartige Schicht ist hierzu am Rand der Basisplatte 3 aufgeklebt.

Die Basisplatte weist auf seiner distalen Seite einen Anschluss 5 für einen Luftzufuhrschlauch 6 auf, an dessen freiem Ende ein Ventil 7 vorgesehen ist. Unterhalb des Anschlusses 5 ist in der Basisplatte 3 eine Durchgangsöffnung 8 vorgesehen, durch die über den Schlauch 6 zugeführte Luft zwischen die Basisplatte 3 und die folienartige Schicht 4 eingebracht werden kann.

Das Ventil 7 ist vorzugsweise so ausgebildet, dass das Verschlussteil des Ventils mit der Düse einer Spritze eingedrückt werden kann, so dass Luft mithilfe der Spritze in das Luftkissen eingebracht oder aus diesem herausgesogen werden kann.

Das Pflaster 2 ist an seiner dem Hals des Patienten zugewandten Seite mit einer Klebeschicht versehen und ist vollumfänglich auf die distale Seite der Basisplatte 3 aufgeklebt. Das Heftpflaster 2 weist ein zentrales, kreisrundes Loch auf, durch das der Anschluss 5 samt Luftzufuhrschlauch 6 vor dem Aufkleben des Pflasters hindurchgesteckt werden können. Das Heftpflaster weist drei in Umfangsrichtung um jeweils 90° zueinander versetzte, kurze Laschen 9, 10, 12 auf, während eine vierte, ebenso um 90° versetzte Lasche 11 deutlich länger ausgebildet ist. Die an gegenüberliegenden Seiten des Luftkissens 1 angeordneten Laschen 9, 10 dienen zur Befestigung des Luftkissens an gegenüberliegenden Seiten des Halses eines Patienten, während die lange Lasche 11 am Hals in Richtung zum Kinn und die gegenüberliegende Lasche 12 am Hals in Richtung zur Brust des Patienten angeklebt wird.

Die Basisplatte 3 und die folienartige Schicht 4 bestehen vorzugsweise aus Kunststoff, wobei für beide Teile verschiedene Kunststoffe verwendet werden können. Als besonders geeignete Kunststoffe haben sich PVC-Kunststoffe und Silikone erwiesen. Auch können insbesondere für die folienartige Schicht gummiartige oder gummihaltige Werkstoffe verwendet werden.

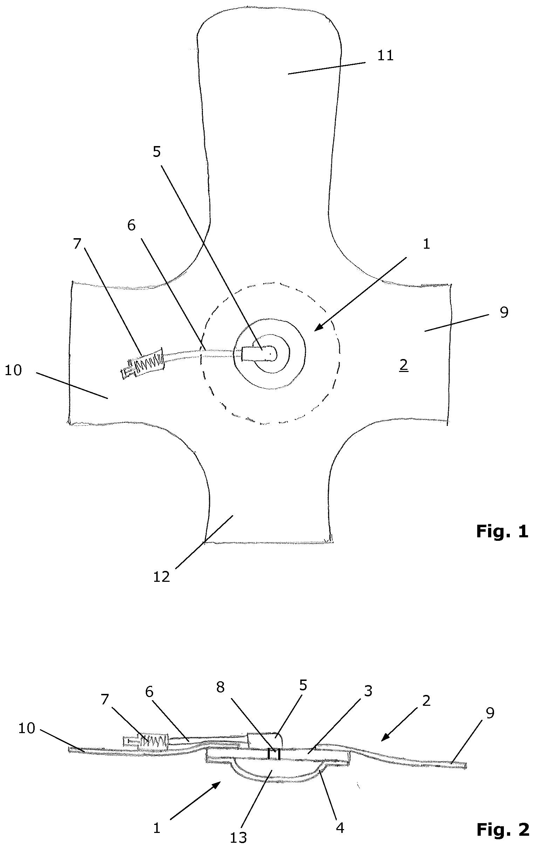

Die Darstellungen der Figuren 1 und 2 sind nicht maßstabsgetreu. Insbesondere sind die Dicken von Pflaster und Basisplatte sowie folienartiger Schicht des Luftkissens zum Zweck der besseren Veranschaulichung deutlich dicker dargestellt.

Im Übrigen ist in Figur 2 das Luftkissen teilweise luftgefüllt dargestellt. Der Raum 13 zwischen der Basisplatte 3 und der folienartigen Schicht 4 kann vollständig verschwinden, wenn er vollständig evakuiert wird und die Schicht 4 dann vollflächig an der Basisplatte 3 anliegt.

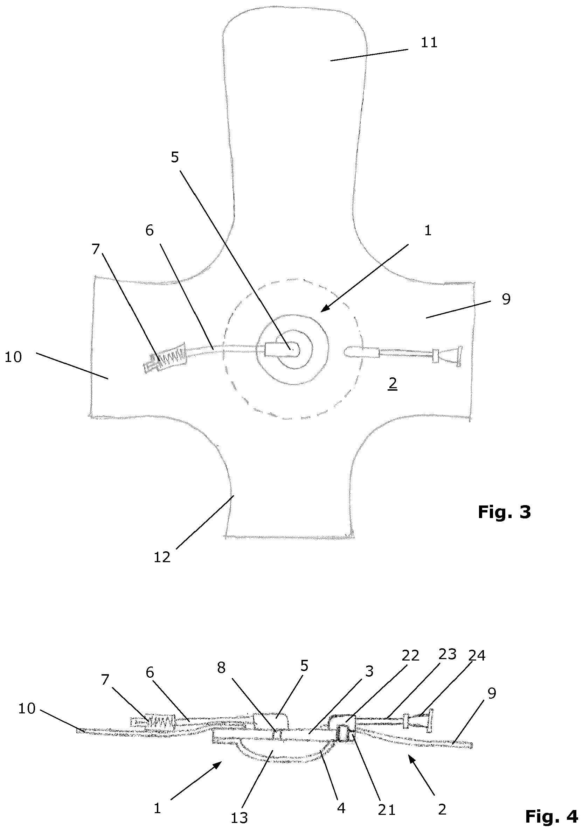

Die in den Figuren 3 und 4 dargestellte Verschlussvorrichtung unterscheidet sich von der in den Figuren 1 und 2 dargestellten lediglich dadurch, dass eine zusätzliche Leitung vorgesehen ist, die von der distalen Seite des Luftkissens zur proximalen Seite des Luftkissens geführt ist. Die Leitung besteht aus einem durch einen flanschartigen Rand (21) des Luftkissens (1) hindurchgeführten Kanalstück (22) und einem sich daran anschließenden Schlauchstück (23). Das Kanalstück (22) erstreckt sich im Wesentlichen senkrecht zum Flanschstück (21) durch dieses hindurch und bildet einen rechtwinklig abgewinkelten Kanal, so dass das Schlauchstück (23) in etwa parallel zum Pflaster (9) geführt werden kann. Am freien Ende des Schlauchstücks (23) ist ein Anschluss (24) zum Verbinden des Schlauchstücks (23) mit einer Vorrichtung zum Erzeugen eines Unterdrucks, insbesondere eine Spritze oder eine Pumpe, vorgesehen.

## Patentansprüche

1. Vorrichtung zum temporären Verschließen eines Trachoestomas eines Patienten mit mindestens einem Mittel zum Halten der Vorrichtung am Hals des Patienten, wobei die Vorrichtung ein aufblasbares Luftkissen (1) aufweist, das mit dem mindestens einen Haltemittel verbunden ist und das geeignet ist, das Tracheostoma eigenständig luftdicht zu verschließen, **dadurch gekennzeichnet, dass** das Luftkissen (1) auf seiner proximalen Seite eine flexible Wandung aufweist, so dass sich die Form des Luftkissens (1) an die Form der an das Tracheostoma angrenzenden Haut anpassen kann.

2. Vorrichtung nach Anspruch 1, **dadurch gekennzeichnet, dass** das Luftkissen (1) einen runden, insbesondere kreisrunden, Querschnitt hat.

3. Vorrichtung nach einem der Ansprüche 1 oder 2, **gekennzeichnet durch** eine mit einem Ventil (7) verschließbare Zuleitung (6) für Luft oder ein anderes Fluid an der distalen Seite des Luftkissens (1) zum Befüllen des Luftkissens (1).

4. Vorrichtung nach einem der Ansprüche 1 bis 3, **dadurch gekennzeichnet, dass** das Luftkissen selbstaufblasbar ausgebildet ist.

5. Vorrichtung einem der Ansprüche 1 bis 4, **dadurch gekennzeichnet, dass** das Luftkissen (1) auf seiner distalen Seite steif ausgebildet ist.

6. Vorrichtung nach einem der Ansprüche 1 bis 5, **dadurch gekennzeichnet, dass** als Haltemittel mindestens ein Heftpflaster (2) vorgesehen ist.

7. Vorrichtung nach Anspruch 6, **dadurch gekennzeichnet, dass** das Heftpflaster (2) an mindestens zwei gegenüberliegenden Seiten des Luftkissens (1) jeweils eine sich vom Luftkissen (1) weg erstreckende Lasche (9, 10, 11, 12) aufweist.

8. Vorrichtung nach Anspruch 7, **gekennzeichnet durch** mindestens drei in Umfangsrichtung des Luftkissens um jeweils 90° versetzt zueinander angeordnete Laschen (9, 10, 11, 12).

9. Vorrichtung nach einem der Ansprüche 1 bis 8, **gekennzeichnet durch** mindestens eine Leitung, die im Bereich des Luftkissens (1) von der distalen Seite der Vorrichtung zu ihrer proximalen Seite geführt ist, und mit der ein Fluid, insbesondere Wundsekret, von der proximalen Seite der Vorrichtung abgeführt werden kann.

10. Vorrichtung nach Anspruch 9, **dadurch gekennzeichnet, dass** die Leitung mit einem durch einen flanschartigen Rand (24) des Luftkissens (1) hindurchgeführten Kanalstück (22) und einem sich daran anschließenden Schlauchstück (23) gebildet ist.

11. Vorrichtung nach Anspruch 10, **dadurch gekennzeichnet, dass** das Schlauchstück (23) mit einem distalen Ende des Kanalstücks (22) lösbar verbunden werden kann.

12. Vorrichtung nach Anspruch 10 oder 11, **dadurch gekennzeichnet, dass** am freien Ende des Schlauchstücks (23) ein Anschluss (24) zum Verbinden des Schlauchstücks mit einer Vorrichtung zum Erzeugen eines Unterdrucks, insbesondere eine Spritze oder eine Pumpe, vorgesehen ist.

## Claims

1. Device for temporarily closing a patient's tracheostoma with at least one means for holding the device on the patient's neck, wherein the device comprises an inflatable air cushion (1), which is connected to the at least one holding means and which is suitable to airtight seal the tracheostoma independently, **characterised in that** the air cushion (1) contains a flexible wall on its proximal side, so that the form of the air cushion (1) can adapt to the form of the skin adjacent to the tracheostoma.

2. Device according to claim 1, **characterised in that** the air cushion (1) has a round cross section, in particular a circular cross section.

3. Device according to one of the claims 1 or 2, **characterised by** a feed line (6) at the distal side of the air cushion (1) for air or another fluid for filling of the air cushion (1), the feedline being closable by a valve (7).

4. Device according to one of the claims 1 to 3, **characterised in that** the air cushion is self-inflatable.

5. Device according to one of the claims 1 to 4, **characterised in that** the air cushion (1) is rigid on its distal side.

6. Device according to one of the claims 1 to 5, **characterised in that** at least one adhesive plaster (2) is provided as holding means.

7. Device according to claim 6, **characterised in that** the adhesive plaster (2) on at least two opposite sides of the air cushion (1) has a flap (9, 10, 11, 12) respectively extending away from the air cushion (1).

8. Device according to claim 7, **characterised by** at least three flaps (9, 10, 11, 12) arranged with an offset of 90° to each other respectively in circumference direction of the air cushion.

9. Device according to one of claims 1 to 8, **characterised by** at least one conduit, which is guided in the region of the air cushion (1), from the distal side of the device to its proximal side and with which a fluid, in particular ichor, can be removed from the proximal side.

10. Device according to claim 9, **characterised in that** the conduit consists of a drain piece (22), which is led through a flange-like edge (24) of the air cushion (1), and a hose piece (23) connected to the drain piece (22).

11. Device according to claim 10, **characterised in that** the hose piece (23) can be releasably connected to a distal end of the drain piece (22).

12. Device according to claim 10 or 11, **characterised in that** at the free end of the hose piece (23) a connection (24) is provided for connecting the hose piece with a device for generating a negative pressure, in particular a syringe or a pump.

## Revendications

1. Dispositif de fermeture temporaire d'un trachéostome d'un patient, comprenant au moins un moyen destiné à retenir le dispositif sur le cou du patient, le dispositif comprenant un coussin d'air (1) gonflable qui est relié audit au moins un moyen de retenue et qui est adapté à fermer de manière étanche le trachéostome de manière autonome, **caractérisé en ce que** le coussin d'air (1) présente sur sa face proximale une paroi flexible, de sorte que la forme du coussin d'air (1) peut s'adapter à la forme de la peau jouxtant le trachéostome.

2. Dispositif selon la revendication 1, **caractérisé en ce que** le coussin d'air (1) présente une section transversale ronde, en particulier circulaire.

3. Dispositif selon l'une quelconque des revendications 1 ou 2, **caractérisé par** une conduite d'amenée (6) pour l'air ou un autre fluide, pouvant être fermée au moyen d'une valve (7), sur la face distale du coussin d'air (1), pour le remplissage du coussin d'air (1).

4. Dispositif selon l'une quelconque des revendications 1 à 3, **caractérisé en ce que** le coussin d'air est auto-gonflable.

5. Dispositif selon l'une quelconque des revendications 1 à 4, **caractérisé en ce que** le coussin d'air (1) est rigide sur sa face distale.

6. Dispositif selon l'une quelconque des revendications 1 à 5, **caractérisé en ce qu'**au moins un sparadrap (2) fait office de moyen de retenue.

7. Dispositif selon la revendication 6, **caractérisé en ce que** le sparadrap (2) présente sur au moins deux faces opposées du coussin d'air (1) respectivement une languette (9, 10, 11, 12) s'étendant à distance du coussin d'air (1).

8. Dispositif selon la revendication 7, **caractérisé par** au moins trois languettes (9, 10, 11, 12) décalées les unes des autres de respectivement 90° dans la direction périphérique du coussin d'air.

9. Dispositif selon l'une quelconque des revendications 1 à 8, **caractérisé par** au moins une conduite qui est guidée dans la zone du coussin d'air (1) de la face distale du dispositif à sa face proximale, et au moyen de laquelle un fluide, en particulier les exsudats d'une plaie, peut être évacué de la face proximale du dispositif.

10. Dispositif selon la revendication 9, **caractérisé en ce que** la conduite est formée d'une portion de canal (22) guidée à travers un bord du type bride (24) du coussin d'air (1) et d'une portion de tuyau flexible (23) située dans le prolongement de ladite portion de canal.

11. Dispositif selon la revendication 10, **caractérisé en ce que** la portion de tuyau flexible (23) peut être reliée de manière libérable à une extrémité distale du le portion de canal (22).

12. Dispositif selon la revendication 10 ou 11, **caractérisé en ce qu'**à l'extrémité libre de la portion de tuyau flexible (23) est prévu un raccord (24) destiné à relier la portion de tuyau flexible à un dispositif de production d'un vide, en particulier une seringue ou une pompe.
